(19) Europäisches Patentamt
European Patent Office
Office européen des brevets

(11) EP 0 485 544 B1

(12) **EUROPÄISCHE PATENTSCHRIFT**

(45) Veröffentlichungstag und Bekanntmachung des Hinweises auf die Patenterteilung:
**16.10.1996 Patentblatt 1996/42**

(21) Anmeldenummer: **91908263.6**

(22) Anmeldetag: **30.04.1991**

(51) Int. Cl.$^6$: **A61K 7/13**

(86) Internationale Anmeldenummer:
**PCT/EP91/00824**

(87) Internationale Veröffentlichungsnummer:
**WO 91/18583 (12.12.1991 Gazette 1991/28)**

# (54) OXIDATIONSHAARFÄRBEMITTEL MIT EINEM GEHALT AN 3-AMINO-PHENOL-DERIVATEN, VERFAHREN ZUM OXIDATIVEN FÄRBEN VON HAAREN SOWIE NEUE 3-AMINOPHENOL-DERIVATE

OXIDATIVE HAIR DYE CONTAINING 3-AMINOPHENOL DERIVATIVES, PROCESS FOR OXIDATIVE DYEING OF HAIR AND NEW 3-AMINOPHENOL DERIVATIVES

COLORANTS D'OXYDATION POUR CHEVEUX, RENFERMANT DES DERIVES 3-AMINOPHENOLS, PROCEDE DE TEINTURE OXYDANTE DES CHEVEUX ET NOUVEAUX DERIVES 3-AMINOPHENOLS

(84) Benannte Vertragsstaaten:
**DE ES FR GB IT**

(30) Priorität: **31.05.1990 DE 4017516**

(43) Veröffentlichungstag der Anmeldung:
**20.05.1992 Patentblatt 1992/21**

(73) Patentinhaber: **Wella Aktiengesellschaft**
**D-64295 Darmstadt (DE)**

(72) Erfinder:
- **CLAUSEN, Thomas**
  **D-6146 Alsbach (DE)**
- **BALZER, Wolfgang, R.**
  **D-6146 Alsbach (DE)**
- **KELLER, Helmut**
  **D-6100 Darmstadt (DE)**

(56) Entgegenhaltungen:
**EP-A- 0 211 238**
**EP-A- 0 215 560**
**EP-A- 0 241 716**
**EP-A- 0 307 777**
**DE-A- 3 827 221**

Bemerkungen:
Die Akte enthält technische Angaben, die nach dem Eingang der Anmeldung eingereicht wurden und die nicht in dieser Patentschrift enthalten sind.

EP 0 485 544 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.13.8/3.4

## Beschreibung

Gegenstand der Erfindung sind ein Mittel und ein Verfahren zur oxidativen Färbung von Haaren auf der Basis von 3-Aminophenol-Derivaten als Kupplersubstanzen sowie neue 3-Aminophenol-Derivate.

Oxidationshaarfarbstoffe haben auf dem Gebiet der Haarfärbung eine wesentliche Bedeutung erlangt. Die Färbung entsteht im Haarschaft durch Reaktion bestimmter Entwicklersubstanzen mit geeigneten Kupplersubstanzen in Gegenwart eines Oxidationsmittels.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Für die erzielbaren Haarfärbungen wird eine gute Licht-, Dauerwell-, Säure- und Reibechtheit gefordert. Auf jeden Fall aber müssen solche Haarfärbungen ohne Einwirkung von Licht, chemischen Mitteln und Reibung über einen Zeitraum von mindestens 4 bis 6 Wochen stabil bleiben. Weiterhin ist es erforderlich, daß durch Kombination geeigneter Entwickler-und Kupplerkomponenten eine breite Palette unterschiedlicher Farbnuancen erzeugt werden kann.

Als Entwicklersubstanzen werden vor allem 1,4-Diaminobenzol, 2,5-Diaminotoluol, 3-Methyl-4-aminophenol und p-Aminophenol eingesetzt. Die bevorzugt verwendeten Kupplersubstanzen sind Resorcin, 4-Chlorresorcin, 2-Methylresorcin, 2,4-Diaminoanisol, m-Phenylendiamin, 1-Naphthol und m-Aminophenol.

Zur Erzeugung von Naturtönen werden bevorzugt Resorcin und m-Aminophenol als Kupplersubstanzen in Verbindung mit p-Phenylendiamin oder 2,5-Diaminotoluol als Entwicklersubstanzen eingesetzt. Dabei wird der durch Resorcin hervorgerufene Gelbstich durch Zusatz von m-Aminophenol kompensiert, so daß wärmere Farbtöne erhalten werden. Für den Einsatz in Oxidationshaarfarbmitteln zur Erzeugung von Modetönen, insbesondere modischen Rottönen, ist m-Aminophenol als Kupplerkomponente nur bedingt geeignet, da es mit p-Aminophenol oder dessen Derivaten nur zu Färbungen von ungenügender Farintensität führt.

Aus der DE-OS 38 17 710 sind Haarfärbemittel, die m-Aminophenol-Derivate als Kupplersubstanzen enthalten, bekannt. Als Beispiel für ein derartiges Aminophenol- Derivat ist 3-Amino-4-methoxyphenol genannt, das jedoch autoxidabel ist und mit p-Phenylendiaminen als Entwicklersubstanzen schwachblaue bis violette Färbungen ergibt. Mit p-Aminophenolen als Entwicklersubstanzen und 3-Amino-4-methoxyphenol als Kupplerkomponente wird unter den üblichen oxidativen Bedingungen kein Farbstoff gebildet.

Weiterhin ist aus der DE-OS 35 24 329 das 5-Amino-4-chlor-2-methylphenol als Kupplersubstanz für Oxidationshaarfärbemittel bekannt. Das 5-Amino-4-chlor-2-methylphenol führt zu Färbungen im Rotbereich, deren Farbintensität und Rotanteil jedoch nicht befriedigen können.

Weiterhin sind aus der EP-OS 0 241 716 Oxidationshaarfärbemittel bekannt, welche eine spezielle Kombination aus der Kupplersubstanz 4-Amino-2-methylphenol und den Entwicklersubstanzen 4-Amino-3-methylphenol und 1,4-Diaminobenzol und/oder 2,5-Diaminotoluol enthalten, wobei befriedigende Farbergebnisse nur bei Verwendung der angegebenen Entwicklersubstanz-Kupplersubstanz-Kombination erzielt werden. Bei Verwendung einer anderen als der angegebenen Entwicklersubstanz werden hingegen keine zufriedenstellenden Färbungen im Rotbereich erzielt.

Es bestand daher die Aufgabe, Mittel zum oxidativen Färben von Haaren zur Verfügung zu stellen, die eine Kupplersubstanz enthalten, welche in Kombination mit Entwicklersubstanzen, wie zum Beispiel p-Aminophenol oder dessen Derivaten, zu Haarfärbungen von hoher Farbintensität im Rotbereich führt und sich für die Nuancierung modischer Rottöne eignet.

Gegenstand der vorliegenden Patentanmeldung ist daher ein Mittel zum oxidativen Färben von Haaren, auf der Basis einer Entwicklersubstanz-Kupplersubstanz-Kombination, dadurch gekennzeichnet, daß es als Kupplersubstanz mindestens ein 3-Aminophenol-Derivat der allgemeinen Formel

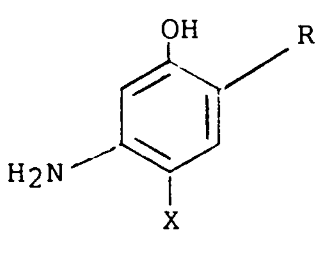

(I),

in der R einen Alkylrest mit 1 bis 4 Kohlenstoffatomen bedeutet und X Fluor oder den Rest $OR^1$ darstellt, wobei $R^1$ einen Alkylrest mit 1 bis 4 Kohlenstoffatomen, einen Monohydroxyalkylrest mit 2 bis 4 Kohlenstoffatomen oder einen Dihydroxyalkylrest mit 3 bis 4 Kohlenstoffatomen bedeutet, oder dessen physiologisch verträgliches wasserlösliches

Salz enthält. Als physiologisch verträgliche, wasserlösliche Salze seien beispielsweise das Chlorid, das Sulfat, das Phosphat, das Propionat, das Lactat und das Citrat genannt.

Besonders geeignet für den Einsatz in diesen Haarfärbemitteln sind die Verbindungen 5-Amino-4-fluor-2-methylphenol, 5-Amino-4-methoxy-2-methylphenol, 5-Amino-4-ethoxy-2-methylphenol, 5-Amino-4-(2'-hydroxyethyl)oxy-2-methylphenol und 5-Amino-2-ethyl-4-fluorphenol.

Gegenstand der vorliegenden Patentanmeldung sind ferner neue 3-Aminophenol-Derivate der allgemeinen Formel

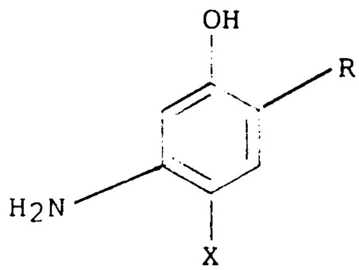

(II),

in der R einen Alkylrest mit 1 bis 4 Kohlenstoffatomen bedeutet und X Fluor oder den Rest $OR^1$ darstellt, wobei $R^1$ einen Alkylrest mit 1 bis 4 Kohlenstoffatomen, einen Monohydroxyalkylrest mit 2 bis 4 Kohlenstoffatomen oder einen Dihydroxyalkylrest mit 3 bis 4 Kohlenstoffatomen bedeutet, unter der Voraussetzung, daß, wenn X Fluor bedeutet, R nicht Methyl ist.

Beispiele für erfindungsgemäße Verbindungen der Formel (II) sind 5-Amino-2-ethyl-4-fluorphenol, 5-Amino-4-methoxy-2-methylphenol, 5-Amino-4-ethoxy-2-methylphenol und 5-Amino-4-(2'-hydroxyethyl)oxy-2-methylphenol.

Die 3-Aminophenol-Derivate der allgemeinen Formel (I) (welche die neuen 3-Aminophenol-Derivate der Formel (II) einschließen) lassen sich in einem allgemein anwendbaren Herstellungsverfahren gemäß dem folgenden Schema, in dem R und $OR^1$ die vorstehend genannte Bedeutung haben sollen, und die Mesylgruppe als Beispiel für eine mögliche Schutzgruppe zu verstehen ist, ausgehend von den 4-Fluor-2-alkylphenolen herstellen.

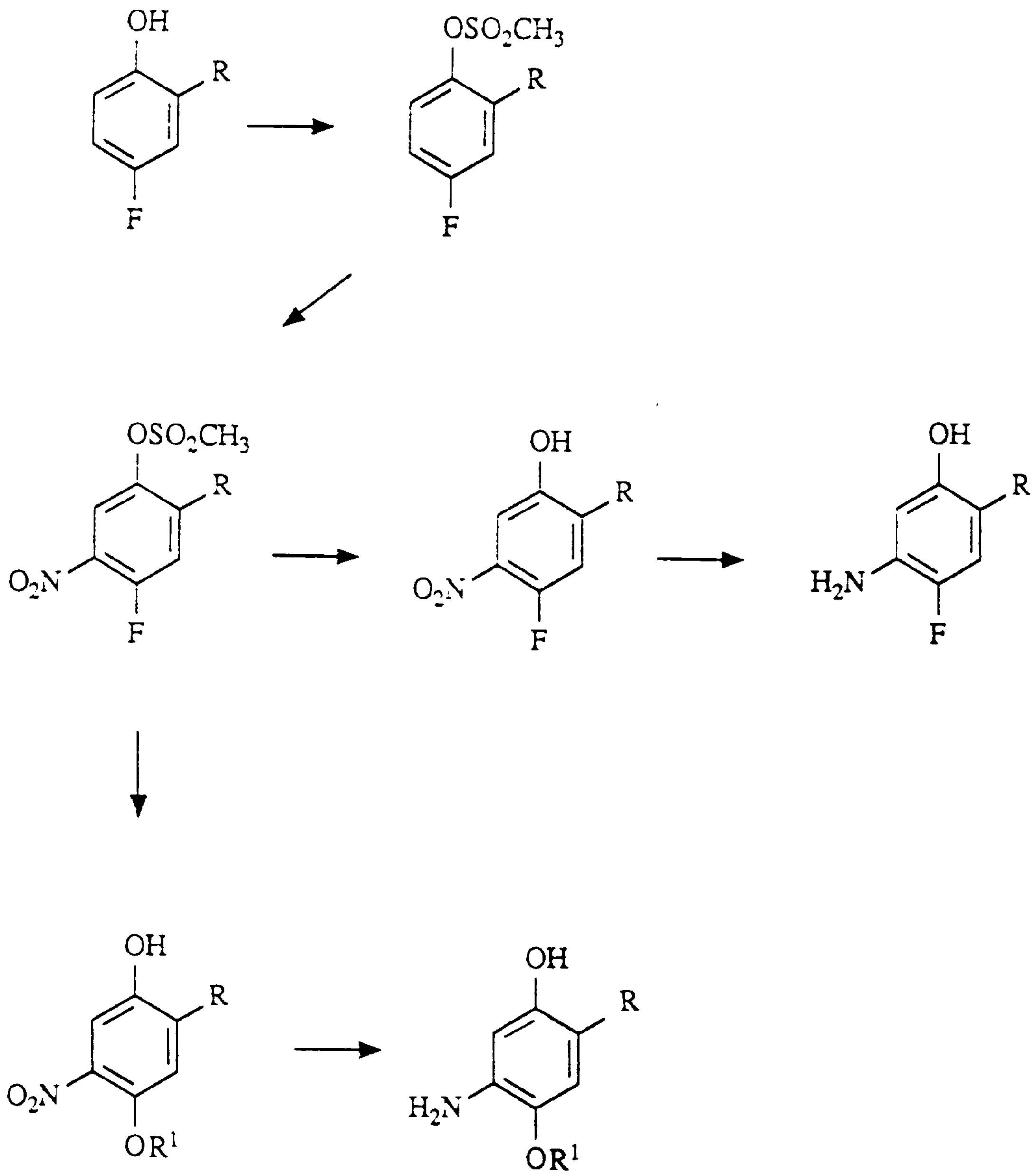

Die 4-Fluor-2-alkylphenole werden zunächst, nach zuvor erfolgter Einführung einer geeigneten Schutzgruppe, zum Beispiel einer Mesylgruppe, für die Hydroxyfunktion mit Salpetersäure in Schwefelsäure nitriert. Die Abspaltung der Schutzgruppe erfolgt im sauren oder alkalischen Milieu, im Fall der Mesylgruppe mit einer starken Base, und ergibt 4-Fluor-5-nitro-2-alkylphenole, die dann zu den 5-Amino-4-fluor-2-alkylphenolen der allgemeinen Formel (I) hydriert werden. Die Hydrierung wird, beispielsweise in alkoholischer Lösung, unter dem Zusatz eines geeigneten Katalysator, zum Beispiel Palladium, durchgeführt. Durch die Umsetzung der 4-Fluor-5-nitro-2-alkylphenole mit einer stark basischen Lösung des entsprechenden Alkohols, beispielsweise Methanol, Ethanol, Ethylenglykol, und einer anschließenden Hydrierung unter Zusatz eines geeigneten Katalysators werden die 5-Amino-4-alkoxy-2-alkylphenole, 5-Amino-4-monohydroxyalkoxy-2-alkylphenole und 5-Amino-4-dihydroxyalkoxy-2-alkylphenole der allgemeinen Formel (I) gewonnen.

Die 3-Aminophenol-Derivate der allgemeinen Formel (I) sind Kupplersubstanzen, die in Kombination mit geeigneten Entwicklersubstanzen zur Färbung von Haaren hervorragend geeignet sind.

Mit den Verbindungen der Formel (I) lassen sich, in Kombination mit Entwicklersubstanzen, wie beispielsweise p-Aminophenol oder dessen Derivaten, intensive Haarfärbungen im Rotbereich erzeugen. Die erzielten Färbungen haben einen deutlich höheren Rotanteil in einen zudem farbsatteren Ton als die mit den üblicherweise eingesetzten Kupplersubstanzen 3-Aminophenol oder 5-Amino-2-methylphenol erzielten Färbungen.

Die sehr guten färberischen Eigenschaften des Haarfärbemittels, das die Verbindungen der allgemeinen Formel (I) enthält, zeigen sich darin, daß dieses Mittel eine Anfärbung von ergrauten, chemisch nicht vorgeschädigten Haaren problemlos und mit guter Deckkraft ermöglicht.

In den Haarfärbemitteln soll die Kupplersubstanz der Formel (I) in einer Menge von 0,01 bis 5,0 Gewichtsprozent, vorzugsweise 0,1 bis 3,0 Gewichtsprozent, enthalten sein.

Die Kupplersubstanz der Formel (I) wird im allgemeinen in etwa molarer Menge, bezogen auf die verwendeten Entwicklersubstanzen, eingesetzt. Wenn sich auch der äquimolare Einsatz als zweckmäßig erweist, so ist es doch nicht nachteilig, wenn die Kupplersubstanz in einem gewissen Überschuß oder Unterschuß zum Einsatz kommt. Es ist ferner nicht notwendig, daß die Entwicklerkomponente und die Kupplerkomponente einheitliche Produkte darstellen, vielmehr kann sowohl die Entwicklerkomponente ein Gemisch von bekannten Entwicklersubstanzen als auch die Kupplerkomponente ein Gemisch der erfindungsgemäßen Verbindungen der allgemeinen Formel (I) mit bekannten Kupplersubstanzen darstellen.

Von den bekannten Kupplersubstanzen kommen als Bestandteil der erfindungsgemäßen Haarfärbemittel vor allem 1-Naphthol, Resorcin, 4-Chlorresorcin, 4,6-Dichlorresorcin, 2-Methylresorcin, 2-Amino-4-(2'-hydroxyethyl)amino-anisol, 2,4-Diaminobenzylalkohol, m-Phenylen-diamin, 2,4-Diamino-1-(difluormethoxy)benzol, 5-Amino-2-methylphenol, 2,4-Diaminophenoxyethanol, m-Aminophenol, 3-Amino-4-chlor-6-methylphenol, 3-Amino-2-methylphenol, 4-Amino-2-hydroxyphenoxyethanol, 4-Hydroxy-1,2-methylendioxybenzol, 4-(2'-Hydroxyethyl)-amino-1,2-methylendioxybenzol, 2,4-Diamino-5-ethoxytoluol, 4-Hydroxyindol, 3-Amino-5-hydroxy-2,6-dimethoxypyridin und 3,5-Diamino-2,6-dimethoxypyridin allein oder im Gemisch oder deren physiologisch verträgliche, wasserlösliche Salze in Betracht.

Außerdem enthalten die Haarfärbemittel Entwicklersubstanzen, insbesondere 1,4-Diaminobenzol, 2,5-Diaminotoluol, 2,5-Diaminobenzylalkohol, 2-(2'-Hydroxyethyl)-1,4-diaminobenzol, 4-Aminophenol, 4-Amino-3-methylphenol, 4-Amino-2-methoxymethylphenol, 4-Amino-2-ethoxymethylphenol sowie Tetraaminopyrimidin oder deren physiologisch verträgliche, wasserlösliche Salze, enthalten sein. Derartige zur Haarfärbung bekannte und übliche Oxidationsfarbstoffe sind unter anderem in dem Buch von E. Sagarin, "Cosmetics, Science and Technologie", Interscience Publishers Inc., New York (1957), Seiten 503 ff. sowie in dem Buch von H. Janistyn, "Handbuch der Kosmetika und Riechstoffe" (1973), Seiten 338 ff. beschrieben. Die Entwicklersubstanzen sind in den beschriebenen Haarfärbemitteln in einer Menge von 0,01 bis 5,0 Gew. %, vorzugsweise 0,1 bis 3,0 Gew. % enthalten.

Die Gesamtmenge der in den hier beschriebenen Haarfärbemitteln, enthaltenen Entwicklersubstanz-Kuppler-Substanz-Kombination soll etwa 0,1 bis 6,0 Gewichtsprozent, vorzugsweise jedoch 0,5 bis 4,0 Gewichtsprozent betragen.

Weiterhin können die Haarfärbemittel auch andere Farbkomponenten, beispielsweise die mit sich selbst kuppelnden Farbstoffe 6-Amino-2-methylphenol, 2-Amino-5-ethoxyphenol und 2-Amino-5-methylphenol sowie ferner übliche direktziehende Farbstoffe, zum Beispiel Triphenylmethanfarbstoffe wie Diamond Fuchsine (C.I. 42 510) und Leather Ruby HF (C.I. 42 520), aromatische Nitrofarbstoffe wie zum Beispiel 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethyl)amino-nitrobenzol und 4-(2'-Hydroxyethylamino)-3-nitrotoluol, 1-(2'-Ureidoethyl)amino -4-nitrobenzol und Azofarbstoffe wie Acid Brown 4 (C.I. 14 805) und Dispersionsfarbstoffe, wie beispielsweise 1,4-Diaminoanthrachinon und 1,4,5,8-Tetraaminoanthrachinon, enthalten.

Weitere geeignete, direkt auf das Haar aufziehende Farbstoffe sind beispielsweise im Buch von J.C. Johnson "Hair Dyes", Noyes Data Corp., Park Ridge, USA (1973), Seiten 3 - 91 und 113 - 139 (ISBN: 0-8155-0477-2) beschrieben.

Die Gesamtmenge der aromatischen Nitrofarbstoffe, der mit sich selbst kuppelnden und der direktziehenden Farbstoffe, der Azo- und Dispersionsfarbstoffe in dem Haarfärbemittel kann 0,1 bis 4,0 Gewichtsprozent betragen.

Selbstverständlich können die Kuppler- und Entwicklersubstanzen sowie die anderen zuvor genannten Farbkomponenten, sofern es Basen sind, auch in Form der physiologisch verträglichen Säureadditionssalze, wie beispielsweise als Hydrochloride oder Sulfate oder - sofern sie aromatische OH-Gruppen besitzen - in Form der Salze mit Basen, zum Beispiel als Alkaliphenolate, in den Haarfärbemitteln eingesetzt werden.

Darüber hinaus können in den Haarfärbemitteln noch weitere übliche kosmetische Zusätze, beispielsweise Antioxidantien wie Ascorbinsäure, Thioglykolsäure oder Natriumsulfit, Parfümöle, Komplexbildner, Netzmittel, Emulgatoren, Verdicker, Pflegestoffe und andere vorhanden sein.

Die Zubereitungsform der hier beschriebenen Haarfärbemittel kann beispielsweise eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätze dar.

Übliche Zusätze für die Haarfärbemittel sind zum Beispiel Lösungsmittel wie Wasser, niedere aliphatische Alkohole, beispielsweise Ethanol, Propanol und Isopropanol sowie mehrwertige Alkohole wie Glykole, Glycerin und 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkohole, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie beispielsweise höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain.

Die erwähnten Bestandteile werden in den für solche Zwecke üblichen Mengen in dem Haarfärbemittel verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt.

Es können aber auch organische Amine, zum Beispiel Monoethanolamin und Triethanolamin, oder auch anorganische Basen wie Natriumhydroxid und Kaliumhydroxid, Verwendung finden.

Gegenstand der Erfindung ist zudem ein Verfahren zur oxidativen Färbung von Haaren, bei dem man das vorstehend beschriebene Haarfärbemittel, unmittelbar vor dem Gebrauch mit einem geeigneten Oxidationsmittel, insbesondere Wasserstoffperoxid, vermischt und dann eine für die Haarfärbung ausreichende Menge, je nach Haarfülle, im allgemeinen 60 bis 200 g, dieses Gemisches auf das Haar aufträgt. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an die Spülung mit einem Shampoo gewaschen und eventuell mit einer schwach organischen Säure, wie zum Beispiel Zitronensäure oder Weinsäure, nachgespült. Anschließend wird das Haar getrocknet.

Als Oxidationsmittel zur Entwicklung der Haarfärbung kommen hauptsächlich Wasserstoffperoxid oder dessen Additionsverbindungen an Harnstoff, Melamin oder Natriumborat in Form der 3 bis 12 %igen, vorzugsweise 6 %igen, wäßrigen Lösungen in Betracht. Wird eine 6 %ige Wasserstoffperoxidlösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet.

Die nachfolgenden Beispiele sollen den Gegenstand der Erfindung näher erläutern ohne ihn auf die Beispiele zu beschränken.

**Herstellunsbeispiele**

**Beispiel 1: 5-Amino-2-ethyl-4-fluorphenol**

1. Stufe: 5-Fluor-2-mesyloxy-ethylbenzol

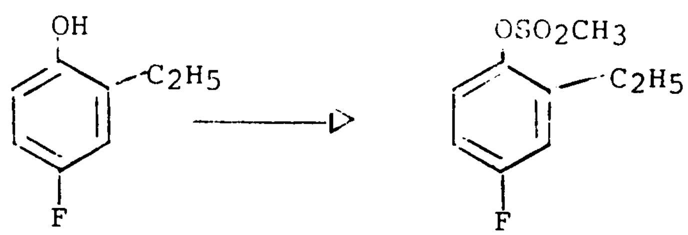

6,27 g (44,79 mmol) 4-Fluor-2-ethylphenol werden in 6,5 ml Pyridin gelöst und unter Rühren werden 6,9 g (60,52 mmol) Mesylchlorid zugetropft. Nach einer Reaktionszeit von 30 min bei 50 Grad Celsius wird das Reaktionsgemisch auf Eis gegeben. Anschließend wird mit Ether extrahiert und mit Wasser gewaschen. Man erhält 8,98 g (41,20 mmol, 92 Prozent der Theorie) eines farblosen Öls mit einem Siedepunkt von 101 Grad Celsius bei 53,33 Pa.

| | |
|---|---|
| $^1$H-NMR ($CDCl_3$): δ = | 1,25 (t; J = 7,5 Hz; 3H; $-CH_2-\underline{CH_3}$) |
| | 2,73 (q; J = 7,5 Hz; 2H; $-\underline{CH_2}-CH_3$) |
| | 3,20 (s, 3H, $-SO_2CH_3$) |
| | 6,92 (ddd, $J_{5,6}$ = 8,9 Hz, $J_{5,F}$ = 8 Hz; $J_{5,3}$ = 3,1 Hz; 1 H; 5-H) |
| | 7,00 (dd, $J_{3,F}$ = 9,1 Hz, $J_{3,5}$ = 3,1 Hz; 1 H; 3-H) |
| | 7,27 ppm (dd; $J_{5,6}$ = 8,9 Hz; $J_{6,F}$ = 4,9 Hz; 1 H; 6-H) |
| MS (70 EV): m/e = | 218 ($M^+$) |

2. Stufe: 5-Fluor-2-mesyloxy-4-nitro-ethylbenzol

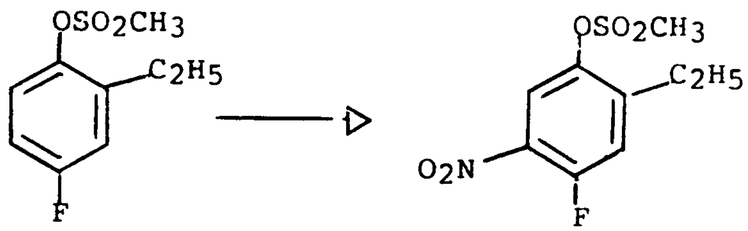

8,72 g (40 mmol) 5-Fluor-2-mesyloxy-etylbenzol werden bei 0 Grad Celsius in 45 ml konzentrierter $H_2SO_4$ gelöst und bei 0 bis 5 Grad Celsius mit 3 ml konzentrierter Salpetersäure versetzt. Nach einer Stunde wird das Reaktionsgemisch auf Eiswasser gegeben, der Niederschlag abgesaugt und mit Wasser neutral gewaschen. Nach dem Umkristallisieren aus Ethanol erhält man 7,9 g (30 mmol, 75 Prozent der Theorie) des Produktes in Form farbloser Kristalle mit einem Schmelzpunkt von 68 Grad Celsius.

| | |
|---|---|
| $^1$H-NMR (DMSO): δ = | 1,29 (t; J = 7,5 Hz; 3 H; $-CH_2-\underline{CH_3}$), |
| | 2,83 (q; J = 7,5 Hz; 2 H; $-C\underline{H_2}-CH_3$), |
| | 3,22 (s; 3 H; $SO_2-C\underline{H_3}$), |
| | 7,24 (d; $J_{3,F}$) = 11,2 Hz; 1 H; 3-H) |
| | 8,04 (d; $J_{6,F}$ = 6,5 Hz; 1 H; 6-H) |
| MS (70 EV): m/e = | 263 ($M^+$) |

3. Stufe: 2-Ethyl-4-fluor-5-nitrophenol

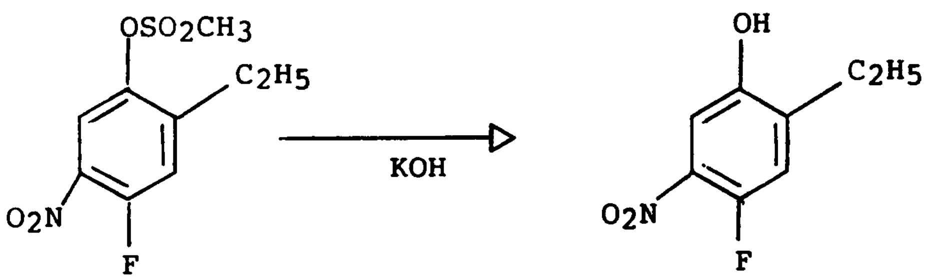


5,26 g (20 mmol) 5-Fluor-2-mesyloxy-4-nitroethylbenzol werden zu einer Lösung von 4 g KOH in 200 ml Wasser gegeben und unter starkem Rühren 30 Minuten auf 60 Grad Celsius erwärmt. Nach dem Abkühlen und Ansäuern mit Salzsäure wird das Reaktionsgemisch auf Eis gegeben und der entstandene Niederschlag abgesaugt. Es werden 2,26 g (12,12 mmol, 61 Prozent der Theorie) des Produktes in Form orange-gelber Kristalle mit einem Schmelzpunkt von 100 bis 102 Grad Celsius erhalten.

| | |
|---|---|
| $^1$H-NMR (DMSO): δ = | 1,26 (t; J = 7,5 Hz; 3 H; $-CH_2-C\underline{H_3}$), |
| | 2,67 (q; J = 7,5 Hz; 2 H; $-\underline{CH_2}-CH_3$), |
| | 5,35 (s; 1H; OH tauscht mit $D_2O$ aus), |
| | 7,06 (d; $J_{3,F}$ = 11,6 Hz, 1 H; 3-H), |
| | 7,49 ppm (d; $J_{6,F}$ = 6,2 Hz; 1 H; 6-H) |
| MS (70 EV): m/e = | 185 ($M^+$) |

4. Stufe: 5-Amino-2-ethyl-4-fluorphenol-Hemisulfat

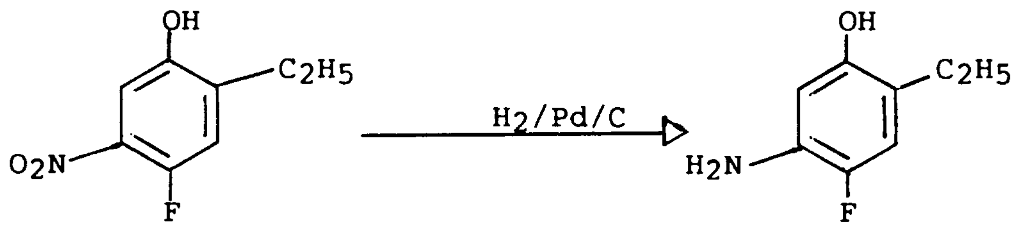


1,95 g (10,53 mmol) 2-Ethyl-4-fluor-5-nitrophenol werden in 20 ml Ethanol gelöst und bei Raumtemperatur über einen Palladium/ Kohlenstoff-Katalysator hydriert. Nachdem die theoretische Menge an Wasserstoff aufgenommen worden ist, wird das Filtrat mit Schwefelsäure angesäuert und das Lösungsmittel in Vakuum abgesaugt. Es werden 1,42 g (9,16 mmol, 87 Prozent der Theorie) des kristallinen Produkts, das bei einer Temperatur von 245 bis 247 Grad Celsius unter Zusetzung schmilzt, erhalten.

$^1$H-NMR (DMSO): δ =

1,08 (t; J = 7,5 Hz; 3 H; $-CH_2-CH_3$),
2,45 (q; J = 7,5 Hz; 2 H; $-CH_2-CH_3$),
6,56 (d; $J_{3,F}$ = 10,5 Hz; 1 H; 3-H),
6,88 (d; $J_{6,F}$ = 6,5 Hz, 1 H; 3-H),
7,8 (s; 2 H; $NH_2$, tauscht mit $D_2O$ aus),
9,3 ppm (s; 1 H; OH; tauscht mit $D_2O$ aus)

MS (70 EV): m/e = 155 ($M^+$)

**Beispiel 2 5-Amino-4-methoxy-2-methylphenol-Hydrochlorid**

1. Stufe: 5-Fluor-2-mesyloxy-toluol

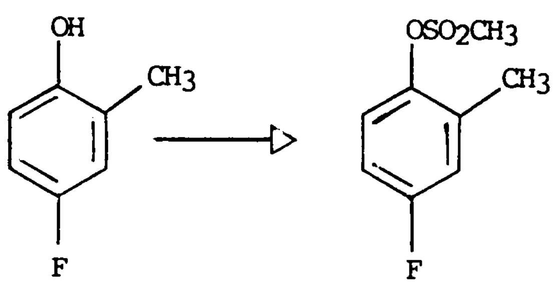

5,64 g (44,76 mmol) 4-Fluor-2methylphenol werden in 6,5 ml Pyridin gelöst. Unter Rühren werden 6,9 g (60,52 mmol) Mesylchlorid zugetropft. Nach einer Reaktionszeit von 30 Minuten bei 50 Grad Celsius, läßt man das Reaktionsgemisch abkühlen, gibt auf Eis und extrahiert mit Einer. Nach dem Waschen mit Wasser und dem Abziehen des Ethers wird der Rückstand im Vakuum destilliert. Es werden 7,3 g (35,78 mmol, 90 Prozent der Theorie) des Produktes mit einem Siedepunkt von 153 bis 155 Grad Celsius bei 1733 Pa und einem Schmelzpunkt von 40 bis 41 Grad Celsius erhalten.

$^1$H-NMR (DMSO): δ = 2,3 (s; 3 H; $Ar-CH_3$)
3,46 (s; 3 H; $-SO_2-CH_3$)
7,13 (m; 1 H; 3-H)

7,24 (d; 1 H; 5-H)
7,37 ppm (m; 1 H; 6-H)

MS (70 eV): m/e = 204 ($M^+$)

2. Stufe: 5-Fluor-2-mesyloxy-4-nitrotoluol

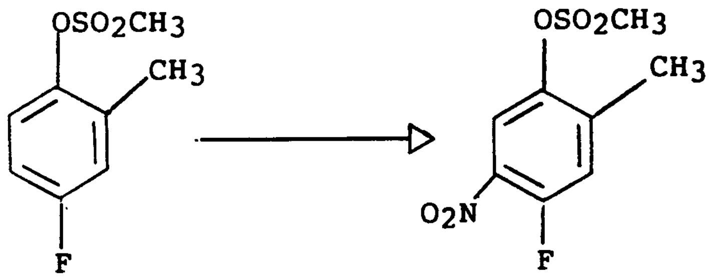

8,16 g (40 mmol) 5-Fluor-2-mesyloxy-toluol werden bei 0 Grad Celsius in 45 ml konzentrierter Schwefelsäure gelöst und bei 0 bis 5 Grad Celsius mit 3 ml konzentrierter Salpetersäure versetzt. Nach 1 Stunde wird das Reaktionsgemisch auf Eiswasser gegeben, der Niederschlag abgesaugt und mit Wasser gewaschen. Nach dem Umkristallisieren aus Ethanol erhält man 7 g (28,4 mmol, 71 Prozent der Theorie) des Produktes in Form gelber Kristalle mit einem Schmelzpunkt von 97 Grad Celsius.

$^1$H-NMR ($CDCl_3$): δ = 2,46 (s; 3 H; $ArCH_3$)
3,31 (s; 3 H; $-SO_2-CH_3$)
7,24 (d; J = 11 Hz; 3-H)
8,02 ppm (d; J = 6 Hz; 6-H)

MS (70 eV): m/e = 249 ($M^+$)

3. Stufe: 4-Methoxy-2-methyl-5-nitrophenol

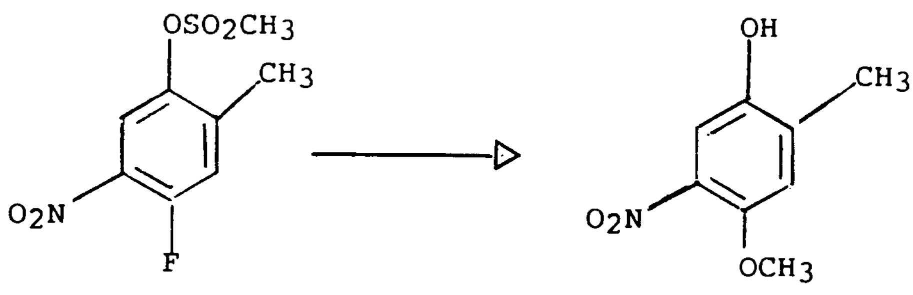

2,3 g (9,24 mmol) 5-Fluor-2-mesyloxy-4-nitrotoluol werden in einer Lösung von 1,3 g KOH in 5 ml Wasser und 50 ml Methanol gelöst und 16 Stunden bei Raumtemperatur gerührt. Der eingeengte Rückstand wird in Dichlormethan aufgenommen und an Kieselgel in Dichlormethan chromatographiert. Es werden 0,84 g (4,25 mmol, 46 Prozent der Theorie) des Produktes in Form dunkelgelber Kristalle mit einem Schmelzpunkt von 82 bis 84 Grad Celsius erhalten.

$^1$H-NMR ($CDCl_3$): δ = 2,32 (s; 3 H; $ArCH_3$)
3,91 (s; 3 H; $O-CH_3$)
5,33 (s (breit); 1 H; OH tauscht mit $D_2O$ aus)

6,87 (s; 1 H; 3-H)
7,41 ppm (s; 1 H; 6-H)

MS (70 eV): m/e = 183 ($M^+$)

4. Stufe: 5-Amino-4-methoxy-2-methylphenol-Hydrochlorid

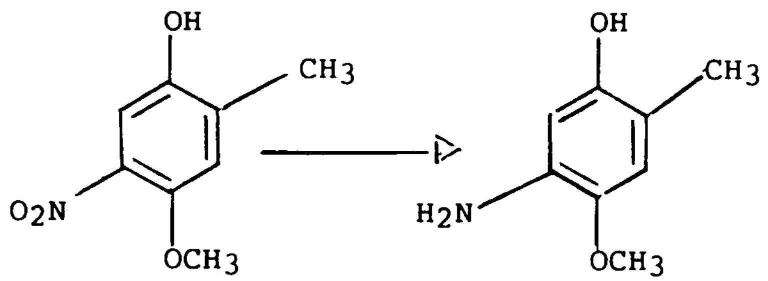

1,8 g (9,84 mmol) 4-Methoxy-2-methyl-5-nitrophenol werden in 20 ml Ethanol gelöst und bei Raumtemperatur über Palladium/ Kohlenstoff hydriert. Nachdem die theoretische Menge an Wasserstoff aufgenommen wurde, wird der Katalysator abfiltriert und das Filtrat mit Salzsäure angesäuert. Nach dem Abziehen des Lösungsmittels werden 1,35 g (8,82 mmol, 90 Prozent der Theorie) des Produktes in Form rötlich-brauner Kristalle, die bei 248 Grad Celsius unter Zersetzung schmelzen, erhalten.

$^1$H-NMR ( $D_6$ -DMSO): δ = 2,1 (s; 3 H; Ar-$CH_3$)
3,76 (s; 3 H; $OCH_3$)
6,94 (s; 1 H; 3-H)
7,01 (s; 1 H; 6-H)
9,5 (s; 1 H; OH; tauscht mit $D_2O$ aus)
10,10 - 10,03 ppm
(s; 2 H; $NH_2$; tauscht mit $D_2O$ aus)

MS (70 eV): m/e = 153 ($M^+$)

**Beispiel 3: 5-Amino-4-ethoxy-2-methylphenol**

1. Stufe und 2. Stufe: siehe **Beispiel 2**

3. Stufe: 4-Ethoxy-2-methyl-5-nitrophenol

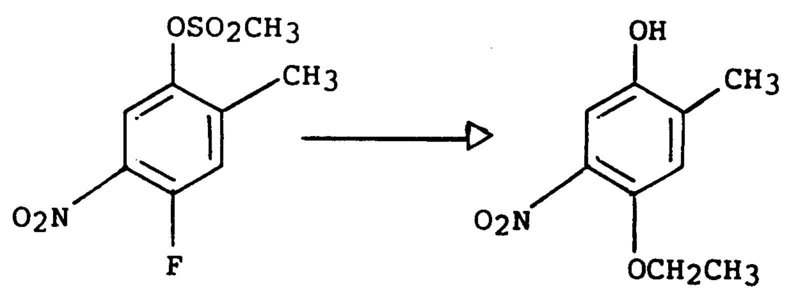

2,3 g (9,24 mmol) 5-Fluor-2-mesyloxy-4-nitrotoluol werden in einer Lösung von 1,3 g KOH in 5 ml Wasser und 50 ml

Ethanol gelöst und 16 Stunden bei Raumtemperatur gerührt. Der eingeengte Rückstand wird in Dichlormethan aufgenommen und an Kieselgel in Dichlormethan chromatographiert.

Es werden 0,6 g (3,01 mmol, 31 Prozent der Theorie) des Produktes in Form dunkelgelber Kristalle mit einem Schmelzpunkt von 81 Grad Celsius erhalten.

$^1$H-NMR ($CDCl_3$): δ = 1,42 (t; J = 6,9 Hz; 3 H; $OCH_2$-$\underline{CH_3}$)
2,29 (s; 3 H; $ArCH_3$)
4,11 (q; 3 = J = 6,9 Hz; 2 H, $O\underline{CH_2}$-$CH_3$)
5,39 (s (breit); 1 H; OH; tauscht mit $D_2O$ aus)
6,85 (s; 1 H; 3-H)
7,36 ppm (s; 1 H; 6-H)

MS (70 eV): m/e = 197 ($M^+$)

4. Stufe: 5-Amino-4-ethoxy-2-methylphenol-Hydrochlorid

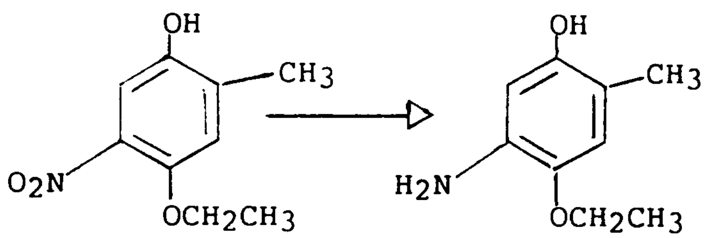

1,94 g (9,84 mmol) 4-Ethoxy-2-methyl-4-nitrophenol werden in 20 ml Ethanol gelöst und bei Raumtemperatur über Palladium/ Kohlenstoff hydriert. Nachdem die theoretische Menge an Wasserstoff aufgenommen wurde, wird der Katalysator abfiltriert und das Filtrat mit Salzsäure angesäuert.

Nach dem Abziehen des Lösungsmittels werden 1,48 g (8,86 mmol, 90 Prozent der Theorie) des Produktes in Form brauner Kristalle, die bei 214 Grad Celsius unter Zersetzung schmelzen, erhalten.

$^1$H-NMR ( $D_6$ -DMSO): δ = 1,32 (t; J = 6,9 ; -O-$CH_2$-$\underline{CH_3}$)
2,10 (s; 3 H; Ar-$CH_3$)
4,02 (q; J = 6,9 Hz; 2 H; -O-$\underline{CH_2}$-$CH_3$)
6,93 (s; 1 H; 6-H)
6,97 (s; 1 H; 3-H)
9,4 (s; 1 H; OH; tauscht mit $D_2O$ aus)
9,89 - 9,99 ppm
(s; 2 H; $NH_2$; tauscht mit $D_2O$ aus)

MS (70 eV): m/e = 167 ($M^+$)

**Beispiel 4: 5-Amino-4-(2'-hydroxyethyl)oxy-2-methylphenol**

1. Stufe und 2. Stufe: siehe **Beispiel 2**

3. Stufe: 4-(2'-Hydroxyethyl)oxy-2-methyl-5-nitrophenol

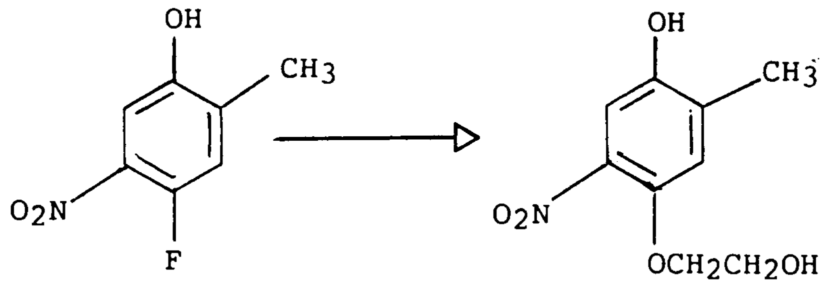

2,3 g (9,24 mmol) 5-Fluor-2-mesyloxy-4-nitrotoluol werden in einer Lösung von 1,3 g KOH in 5 ml Wasser und 50 ml 1,2-Ethandiol gelöst und 16 Stunden bei Raumtemperatur gerührt. Der eingeengte Rückstand wird in Dichlormethan aufgenommen und an Kieselgel in Dichlormethan chromatographiert. Es werden 0,55 g (2,58 mmol, 28 Prozent der Theorie) in Form gelber Kristalle mit einem Schmelzpunkt von 150 Grad Celsius erhalten.

| | |
|---|---|
| $^1$H-NMR ($CDCl_3$): $\delta$ = | 2,19 (s; 3 H, Ar-$CH_3$) |
| | 3,69 (m; 2 H, $OCH_2$-$\underline{CH_2}$-OH) |
| | 4,06 (t; J = 5 Hz; 2 H, $\underline{OCH_2}$-$CH_2$-OH) |
| | 4,83 (s (breit); 1 H; $OCH_2$-$\underline{CH_2}$-OH, tauscht mit $D_2O$ aus) |
| | 7,14 (s; 1 H; 3-H) |
| | 7,26 (s; 1 H; 6-H) |
| | 9,7 ppm (s (breit); 1 H; OH; tauscht mit $D_2O$ aus) |
| MS (70 eV): m/e = | 213 ($M^+$) |

4. Stufe: 5-Amino-4-(2'-hydroxyethyl)oxy-2-methylphenol-Hydrochlorid

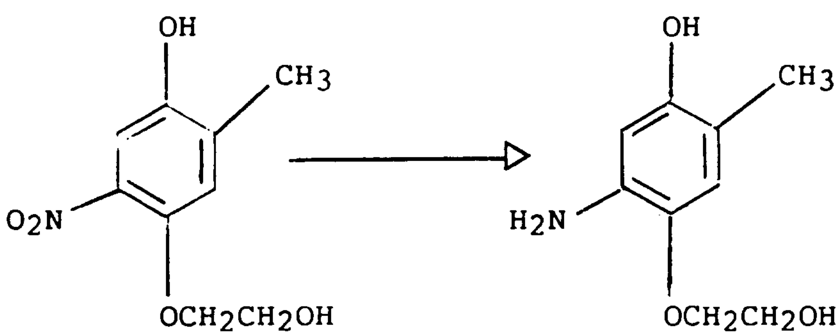

2,1 g (9,84 mmol) 4-(2'-Hydroxyethyl)oxy-2-methyl-5-nitrophenol werden in 20 ml Ethanol gelöst und bei Raumtemperatur über Palladium/Kohlenstoff hydriert. Nachdem die theoretische Menge an Wasserstoff aufgenommen wurde, wird der Katalysator abfiltriert und das Filtrat mit Salzsäure angesäuert. Nach dem Abziehen des Lösungsmittels werden 1,62 g (8,86 mmol, 90 Prozent der Theorie) des Produkts in Form dunkelbrauner Kristalle, die bei 183 bis 185 Grad Celsius unter Zersetzung schmelzen erhalten.

| | |
|---|---|
| $^1H$-NMR ( $D_6$ -DMSO): δ = | 2,11 (s; 3 H, Ar-$CH_3$) |
| | 3,69 (m; 2 H; $OCH_2CH_2OH$ |
| | 3,99 (t, J = 4,6 Hz; 2H; $OCH_2CH_2OH$) |
| | 4,1 - 4,5 (s (breit); 1 H; $OCH_2CH_2OH$ tauscht mit $D_2O$ aus) |
| | 6,93 (s; 1 H; 3-H) |
| | 6,94 (s; 1 H; 6-H) |
| | 9,45 (s (breit); 1 H; OH; tauscht mit $D_2O$ aus) |
| | 10,01 ppm (s (breit); 2 H; $NH_2$, tauscht mit $D_2O$ aus) |
| MS (70 eV): m/e = | 183 ($M^+$) |

Alle in der vorliegenden Anmeldung angegebenen $^1H$-NMR-Spektren (Protonenresonanzspektren) wurden mit einem 300 Megahertz-$^1H$-NMR-Spektrometer (Bruker MW 300) aufgenommen. Die chemischen Verschiebungen sind relativ zur Standardsubstanz Tetramethylsilan (TMS), für die gilt TMS = Oppm, in ppm angeben. Die bei den angegebenen $^1H$-TMS = Oppm, in ppm angeben.

Die bei den angegebenen $^1H$-NMR-Daten verwendeten Abkürzungen sollen die folgenden Bedeutungen haben:

| | |
|---|---|
| s | Singulett |
| d | Dublett |
| t | Triplett |
| q | Quartett |
| m | Multiplett |
| s (breit) | breites Singulett |
| ppm | parts per million, ein Millionstel der Senderleistung |
| J | Kopplungskonstante |
| $CDCl_3$ | Deuterochloroform |
| $D_6$-DMSO | Deuterodimethylsulfoxid |

**Beispiele für Haarfärbemittel**

**Beispiel A: Haarfärbemittel in Gelform**

| | |
|---|---|
| 0,22 g | 4-Aminophenol |
| 0,37 g | 5-Amino-4-fluor-2-methylphenol-Hemisulfat |
| 0,30 g | Ascorbinsäure |
| 15,00 g | Ölsäure |
| 7,00 g | Isopropanol |
| 10,00 g | Ammoniak, 22 %ige wäßrige Lösung |
| 67,11 g | Wasser |
| 100,00 g | |

50 g dieses Haarfärbemittels in Gelform werden unmittelbar vor Anwendung mit 50 g Wasserstoffperoxidlösung (6 %ig) vermischt und dann auf weiße, menschliche Haare auftragen. Man läßt das Gemisch 30 Minuten lang bei 40 Grad Celsius einwirken. Danach wird die Färbemasse mit Wasser ausgespült, das Haar gegebenenfalls shampooniert, mit Wasser gespült und sodann getrocknet. Das Haar ist in einem intensiven, orangen Farbton gefärbt.

**Beispiel B: Haarfärbelösung**

| | |
|---|---|
| 0,80 g | 4-Aminophenol |
| 0,12 g | Resorcin |
| 0,10 g | m-Aminophenol |
| 1,05 g | 5-Amino-4-(2'-hydroxyethyl)oxy-2-methylphenol-Hydrochlorid |
| 0,15 g | 2-Amino-4-(2'-hydroxyethyl)aminoanisol-Hemisulfat |
| 0,05 g | 1-Naphthol |
| 10,00 g | Natriumlaurylalkoholdiglykolethersulfat, 28 %ige wäßrige Lösung |
| 10,00 g | Ammoniak, 22 %ige wäßrige Lösung |
| 77,73 g | Wasser |
| 100,00 g | |

Kurz vor der Anwendung werden 50 g dieser Haarfärbelösung mit 50 g Wasserstoffperoxidlösung (6 %ig) vermischt und auf blonde, menschliche Haare aufgetragen. Man läßt das Gemisch 30 Minuten lang bei 40 Grad Celsius einwirken, spült die Färbemasse sodann mit Wasser aus, shampooniert das Haar gegebenenfalls und trocknet es. Das Haar ist in einem modischen, intensiven goldorangen Farbton gefärbt.

**Beispiel C: Färbemittel in Gelform**

| | |
|---|---|
| 0,48 g | 4-Aminophenol |
| 0,80 g | 2,5-Diaminotoluolsulfat |
| 0,22 g | 2-Amino-4-(2'-hydroxyethyl)amino-anisol-Hemisulfat |
| 0,31 g | 5-Amino-4-methoxy-2-methylphenol-Hydrochlorid |
| 0,02 g | 1-[(2'-Ureidoethyl)amino]-4-nitro-benzol |
| 0,05 g | 2-Nitro-p-phenylendiamin |
| 0,15 g | Natriumsulfit, wasserfrei |
| 2,50 g | Natriumlaurylalkoholdiglykolether-sulfat, 28 %ige wäßrige Lösung |
| 0,80 g | Hydroxyethylcellulose, hochviskos |
| 6,00 g | Ammoniak, 22 %ige wäßrige Lösung |
| 88,67 g | Wasser |
| 100,00 g | |

50 g des vorstehenden Färbemittels in Gelform werden kurz vor der Anwendung mit 50 g Wasserstoffperoxidlösung (6 %ig) vermischt. Man trägt die Mischung auf blonde, menschliche Naturhaare auf und läßt sie 30 Minuten bei 40 Grad Celsius einwirken. Anschließend wird das Haar mit Wasser gespült, gegebenenfalls shampooniert und getrocknet. Das so behandelte Haar hat eine modische, intensive kupferbraune Färbung angenommen.

**Beispiel D: Haarfärbemittel in Gelform**

| | |
|---|---|
| 0,25 g | 4-Amino-3-methylphenol |
| 0,45 g | 5-Amino-4-ethoxy-2-methylphenol-Hydrochlorid |
| 0,30 g | Ascorbinsäure |
| 15,00 g | Ölsäure |
| 7,00 g | Isopropanol |
| 10,00 g | Ammoniak, 22 %ige wäßrige Lösung |
| 67,00 g | Wasser |
| 100,00 g | |

Man vermischt kurz vor dem Gebrauch 50 g dieses Haarfärbemittels in Gelform mit 50 g Wasserstoffperoxidlösung (6 %ig), trägt das Gemisch auf weiße, menschliche Haare auf und läßt es 30 Minuten lang bei 40 Grad Celsius einwirken. Sodann wird das Haar mit Wasser gespült, gegebenenfalls shampooniert und getrocknet. Das Haar ist in einem intensiven roten Farbton gefärbt.

**Beispiel E: Haarfärbelösung**

| | |
|---|---|
| 2,26 g | 4-Aminophenol |
| 0,95 g | 4-Amino-3-methylphenol |
| 3,53 g | 5-Amino-2-ethyl-4-fluorphenol-Hemisulfat |
| 1,28 g | 2-Amino-4-(2'-hydroxyethyl)amino-anisolsulfat |
| 0,50 g | 1-Naphthol |
| 10,00 g | Natriumlaurylalkoholdiglykolether-sulfat, 28 %ige wäßrige Lösung |
| 10,00 g | Ammoniak, 22 %ige wäßrige Lösung |
| 71,48 g | Wasser |
| 100,00 g | |

Kurz vor der Anwendung werden 50 g der vorstehenden Haarfärbelösung mit 50 g Wasserstoffperoxidlösung (6 %ig) vermischt. Man trägt das Gemisch auf menschliche, blonde Naturhaare auf und läßt es bei 40 Grad Celsius 30 Minuten lang einwirken. Sodann wird das Haar mit Wasser gespült, gegebenenfalls shampooniert und getrocknet. Das Haar hat eine intensive rot-braune Färbung angenommen.

Alle in der vorliegenden Anmeldung angegebenen Prozentzahlen stellen, sofern nicht anders vermerkt, Gewichtsprozente dar.

**Patentansprüche**

1. Mittel zur oxidativen Färbung von Haaren auf der Basis einer Entwicklersubstanz-Kupplersubstanz-Kombination, dadurch gekennzeichnet, daß es als Kupplersubstanz mindestens ein 3-Aminophenol-Derivat der allgemeinen Formel

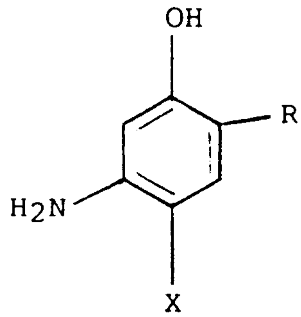

(I),

in der R einen Alkylrest mit 1 bis 4 Kohlenstoffatomen bedeutet und X Fluor oder den Rest $OR^1$ darstellt, wobei $R^1$ einen Alkylrest mit 1 bis 4 Kohlenstoffatomen, einen Monohydroxyalkylrest mit 2 bis 4 Kohlenstoffatomen oder einen Dihydroxyalkylrest mit 3 bis 4 Kohlenstoffatomen bedeutet, oder dessen physiologisch verträgliches wasserlösliches Salz enthält.

2. Mittel nach Anspruch 1, dadurch gekennzeichnet, daß die Kupplersubstanz der Formel (I) ausgewählt ist aus 5-Amino-4-fluor-2-methylphenol, 5-Amino-4-methoxy-2-methylphenol, 5-Amino-4-ethoxy-2-methylphenol, 5-Amino-4-(2'-hydroxyethyl)oxy-2-methylphenol und 5-Amino-2-ethyl-4-fluorphenol.

3. Mittel nach Anspruch 1 oder 2, dadurch gekennzeichnet, daß die Kupplersubstanz der Formel (I) in einer Menge von 0,01 bis 5,0 Gewichtsprozent enthalten ist.

4. Mittel nach einem der Ansprüche 1 bis 3, dadurch gekennzeichnet, daß es mindestens eine der Kupplersubstanzen 1-Naphthol, Resorcin, 4-Chlorresorcin, 4,6-Dichlorresorcin, 2-Methylresorcin, 2-Amino-4-(2'-hydroxyethylamino)-anisol, 5-Amino-2-methylphenol, 2,4-Diaminophenoxyethanol, 4-Amino-2-hydroxy-phenoxyethanol, m-Aminophenol, 3-Amino-4-chlor-6-methylphenol, 3-Amino-2-methylphenol, 4-Hydroxy-1,2-methylendioxybenzol, 4-(2'-Hydroxyethylamino)-1,2-methylendioxybenzol, 2,4-Diamino-5-ethoxytoluol, 2,4-Diaminobenzylalkohol, m-Phenylendiamin, 4-Hydroxyindol, 3-Amino-5-hydroxy-2,6-dimethoxypyridin und 3,5-Diamino-2,6-dimethoxypyridin oder deren physiologisch verträgliches, wasserlösliches Salz enthält.

5. Mittel nach einem der Ansprüche 1 bis 4, dadurch gekennzeichnet, daß es mindestens eine der Entwicklersubstanzen 1,4-Diaminobenzol, 2,5-Diaminotoluol, 2,5-Diaminobenzylalkohol, 2-(2'-Hydroxyethyl)-1,4-diaminobenzol, 4-Aminophenol, 4-Amino-3-methylphenol, 4-Amino-2-methoxymethylphenol, 4-Amino-2-ethoxymethylphenol und Tetraaminopyrimidin oder deren physiogisch verträgliches, wasserlösliches Salz enthält.

6. Mittel nach einem der Ansprüche 1 bis 5, dadurch gekennzeichnet, daß die Gesamtmenge der darin enthaltenen Entwicklersubstanz-Kupplersubstanz-Kombination 0,1 bis 6,0 Gewichtsprozent beträgt.

7. Mittel nach einem der Ansprüche 1 bis 6, dadurch gekennzeichnet, daß es 6-Amino-2-methylphenol, 2-Amino-5-methylphenol oder 2-Amino-5-ethoxyphenol enthält.

8. Mittel nach einem der Ansprüche 1 bis 7, dadurch gekennzeichnet, daß es mindestens einen der direkt auf das Haar aufziehenden Farbstoffe Diamond Fuchsine (C.I. 42 510), Leather Ruby HF (C.I. 42 520), 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethylamino)-nitrobenzol, 4-(2'-Hydroxyethylamino)-3-nitrotoluol, 1-(2'-Ureidoethyl)-amino -4-nitrobenzol, Acid Brown 4 (C.I. 14 805), 1,4-Diaminoanthrachinon und 1,4,5,8-Tetraaminoanthrachinon oder deren physiologisch verträgliche Salze enthält.

**9.** 3-Aminophenol-Derivat der allgemeinen Formel

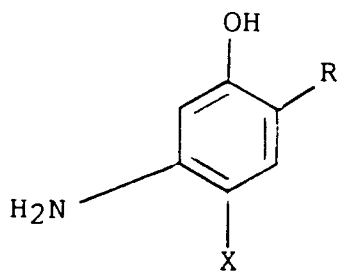

(II),

in der R einen Alkylrest mit 1 bis 4 Kohlenstoffatomen bedeutet und X Fluor oder den Rest $OR^1$ darstellt, wobei $R^1$ einen Alkylrest mit 1 bis 4 Kohlenstoffatomen, einen Monohydroxyalkylrest mit 2 bis 4 Kohlenstoffatomen oder einen Dihydroxyalkylrest mit 3 bis 4 Kohlenstoffatomen bedeutet, unter der Voraussetzung, daß, wenn X Fluor bedeutet, R nicht Methyl ist.

**10.** 5-Amino-4-methoxy-2-methylphenol

**11.** 5-Amino-4-ethoxy-2-methylphenol

**12.** 5-Amino-4-(2'-hydroxyethyl)oxy-2-methylphenol

**13.** 5-Amino-2-ethyl-4-fluorphenol

**14.** Verfahren zur oxidativen Färbung von Haaren, dadurch gekennzeichnet, daß man eine zur Haarfärbung ausreichende Menge eines Haarfärbemittels nach einem der Ansprüche 1 bis 8 unmittelbar vor dem Gebrauch mit einem Oxidationsmittel, insbesondere Wasserstoffperoxid, vermischt und sodann auf die Haare aufbringt, 10 bis 45 Minuten lang bei einer Temperatur von 15 bis 50 Grad Celsius einwirken läßt, anschließend die Haare mit Wasser ausspült, und sodann trocknet.

## Claims

**1.** Agent for the oxidative dyeing of hair based on a developer substance - coupler substance combination, characterised in that it contains as coupler substance at least one 3-aminophenol derivative of the general formula

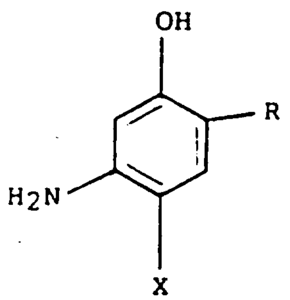

(I),

in which R is an alkyl residue with 1 to 4 carbon atoms and X is fluorine or the residue $OR^1$, $R^1$ being an alkyl residue with 1 to 4 carbon atoms, a monohydroxyalkyl residue with 2 to 4 carbon atoms or a dihydroxyalkyl residue with 3 to 4 carbon atoms, or a physiologically-compatible water-soluble salt thereof.

**2.** Agent according to Claim 1, characterised in that the coupler substance of formula (I) is selected from 5-amino-4-fluoro-2-methylphenol, 5-amino-4-methoxy-2-methylphenol, 5-amino-4-ethoxy-2-methylphenol, 5-amino-4-(2'-hydroxyethyl)oxy-2-methylphenol and 5-amino-2-ethyl-4-fluorophenol.

3. Agent according to Claim 1 or 2, characterised in that the coupler substance of formula (I) is present in an amount of 0.01 to 5.0 weight percent.

4. Agent according to any one of Claims 1 to 3, characterised in that it contains at least one of the coupler substances 1-naphthol, resorcinol, 4-chlororesorcinol, 4,6-dichlororesorcinol, 2-methylresorcinol, 2-amino-4-(2'-hydroxyethylamino)-anisole, 5-amino-2-methylphenol, 2,4-diaminophenoxyethanol, 4-amino-2-hydroxy-phenoxyethanol, m-aminophenol, 3-amino-4-chloro-6-methylphenol, 3-amino-2-methylphenol, 4-hydroxy-1,2-methylenedioxybenzene, 4-(2'-hydroxyethylamino)-1,2-methylenedioxybenzene, 2,4-diamino-5-ethoxytoluene, 2,4-diaminobenzyl alcohol, m-phenylenediamine, 4-hydroxyindole, 3-amino-5-hydroxy-2,6-dimethoxypyridine and 3,5-diamino-2,6-dimethoxypyridine or a physiologically compatible water-soluble salt thereof.

5. Agent according to any one of Claims 1 to 4, characterised in that it contains at least one of the developer substances 1,4 diaminobenzene, 2,5-diamino-toluene, 2,5-diaminobenzyl alcohol, 2-(2'-hydroxyethyl)-1,4-diaminobenzene, 4-aminophenol, 4-amino-3-methylphenol, 4-amino-2-methoxymethylphenol, 4-amino-2-ethoxymethylphenol and tetraaminopyrimidine or a physiologically compatible water-soluble salt thereof.

6. Agent according to any one of Claims 1 to 5, characterised in that the total amount of developer substance - coupler substance present in the agent is 0.1 to 6.0 weight percent.

7. Agent according to any one of Claims 1 to 6, characterised in that it contains 6-amino-2-methylphenol, 2-amino-5-methylphenol or 2-amino-5-ethoxyphenol.

8. Agent according to any one of Claims 1 to 7, characterised in that it contains at least one of the following dyes which acts directly on the hair: Diamond Fuchsine (C.I. 42 510), Leather Ruby HF (C.I. 42 520), 2-nitro-1,4-diaminobenzene, 2-amino-4-nitrophenol, 2-amino-5-nitrophenol, 2-amino-4,6-dinitrophenol, 2-amino-5-(2'-hydroxyethylamino)-nitrobenzene, 4-(2'-hydroxyethylamino)-3-nitrotoluene, 1-(2'-ureidoethyl)-amino-4-nitrobenzene, Acid Brown 4 (C.I. 14 805), 1,4-diaminoanthraquinone and 1,4,5,8-tetraaminoanthraquinone or physiologically compatible salts thereof.

9. 3-aminophenol derivative of the general formula

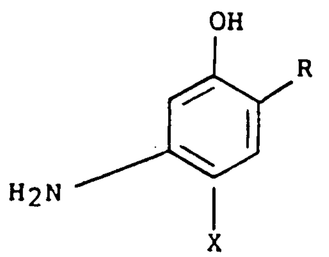

(II),

in which R is an alkyl residue with 1 to 4 carbon atoms and X is fluorine or the residue $OR^1$, $R^1$ being an alkyl residue with 1 to 4 carbon atoms, a monohydroxyalkyl residue with 2 to 4 carbon atoms or a dihydroxyalkyl residue with 3 to 4 carbon atoms, provided that R is not methyl if X is fluorine.

10. 5-amino-4-methoxy-2-methylphenol.

11. 5-amino-4-ethoxy-2-methylphenol.

12. 5-amino-4-(2'-hydroxyethyl)oxy-2-methylphenol.

13. 5-amino-2-ethyl-4-fluorophenol.

14. Process for the oxidative dyeing of hair, characterised in that an amount of a hair dyeing agent according to any one of Claims 1 to 8 sufficient for dyeing the hair is mixed immediately before use with an oxidising agent, especially hydrogen peroxide, and is then applied to the hair, left to act for 10 to 45 minutes at a temperature of 15 to 50°C, the hair is subsequently rinsed with water and then dried.

## Revendications

1. Produit de coloration des cheveux par oxydation à base d'une combinaison développeur-copulateur, caractérisé en ce qu'il renferme, comme copulateur, au moins un dérivé de 3-aminophénol répondant à la formule générale

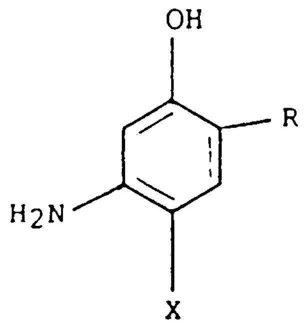

(I),

dans laquelle R représente un reste alkyle comportant 1 à 4 atomes de carbone et X le fluor ou le reste $OR^1$, $R^1$ représentant un reste alkyle comportant 1 à 4 atomes de carbone, un reste monohydroxyalkyle comportant 2 à 4 atomes de carbone ou un reste dihydroxyalkyle comportant 3 à 4 atomes de carbone, ou son sel soluble dans l'eau physiologiquement compatible.

2. Produit selon la revendication 1, caractérisé en ce que le copulateur de formule (I) est choisi parmi les 5-amino-4-fluoro-2-méthylphénol, 5-amino-4-méthoxy-2-méthylphénol, 5-amino-4-éthoxy-2-méthylphénol, 5-amino-4-(2'-hydroxyéthyl)oxy-2-méthylphénol et 5-amino-2-éthyl-4-fluorophénol.

3. Produit selon la revendication 1 ou 2, caractérisé en ce que le copulateur de formule (I) est contenu dans une proportion de 0,01 à 5,0 % en poids.

4. Produit selon l'une des revendications 1 à 3, caractérisé en ce qu'il contient au moins l'un des copulateurs suivants : 1-naphtol, la résorcine, la 4-chlororésorcine, la 4,6-dichlororésorcine, la 2-méthylrésorcine, le 2-amino-4-(2'-hydroxyéthylamino)anisole, le 5-amino-2-méthylphénol le 2,4-diaminophénoxyéthanol, le 4-amino-2-hydroxyphénoxyéthanol le m-aminophénol, le 3-amino-4-chloro-6-méthylphénol le 3-amino-2-méthylphénol le 4-hydroxy-1,2-méthylènedioxybenzène, le 4-(2'-hydroxyéthylamino)-1,2-méthylène-dioxy-benzène, le 2,4-diamino-5-éthoxytoluène, l'alcool 2,4-diaminobenzylique, la m-phénylène-diamine, le 4-hydroxy-indole, la 3-amino-5-hydroxy-2,6-diméthoxypyridine et la 3,5-diamino-2,6-diméthoxypyridine, ou leur sel soluble dans l'eau physiologiquement compatible.

5. Produit selon l'une des revendications 1 à 4, caractérisé en ce qu'il contient l'un au moins des développeurs suivants : le 1,4-diaminobenzène, le 2,5-diaminotoluène, l'alcool 2,5-diaminobenzylique, le 2-(2'-hydroxyéthyl)-1,4-diaminobnezène, le 4-aminophénol, le 4-amino-3-méthylphénol, le 4-amino-2-méthoxyméthylphénol, le 4-amino-2-éthoxyméthylphyénol et la tétra-aminopyrimidine ou leurs sels soluble dans l'eau physiologiquement compatible.

6. Produit selon l'une des revendications 1 à 5, caractérisé en ce que la proportion totale de la combinaison développeur-copulateur qui y est contenue est de 0,1 à 6,0 % en poids.

7. Produit selon l'une des revendications 1 à 6, caractérisé en ce qu'il contient du 6-amino-2-méthylphénol, du 2-amino-5-méthylphénol ou du 2-amino-5-éthoxyphénol.

8. Produit selon l'une des revendications 1 à 7, caractérisé en ce qu'il renferme l'un au moins des colorants à montée directe sur les cheveux suivants : Diamond Fuchsine (I.C. 42 510), Leather Ruby HF (I.C. 42 520), le 2-nitro-1,4-diaminobenzène, le 2-amino-4-nitrophénol le 2-amino-5-nitrophénol le 2-amino-4,6-dinitrophénol, le 2-amino-5-(2'-hydroxyéthylamino)nitrobenzène, le 4-(2'-hydroxyéthylamino)-3-nitrotoluène, le 1-(2'-uréidoéthyl)-amino-4-nitrobenzène, Acid Brown (I.C. 14 805), la 1,4-diaminoanthraquinone et la 1,4,5,8-tétra-amino-anthraquinone, ou leurs sels physiologiquement compatibles.

**9.** Dérivé du 3-aminophénol répondant à la formule générale

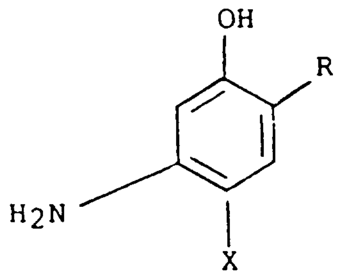

(II),

dans laquelle R représente un reste alkyle comportant 1 à 4 atomes de carbone et X représente le fluor ou le reste $OR^1$, $R^1$ représentant un reste alkyle comportant 1 à 4 atomes de carbone, un reste monohydroxyalkyle comportant 2 à 4 atomes de carbone ou un reste dihydroxyalkyle comportant 3 à 4 atomes de carbone, à condition que, si X représente le fluor, R ne soit pas un méthyle.

**10.** 5-amino-4-méthoxy-2-méthylphénol.

**11.** 5-amino-4-éthoxy-2-méthylphénol.

**12.** 5-amino-4-(2'-hydroxyéthyl)oxy-2-méthylphénol.

**13.** 5-amino-2-éthyl-4-fluorophénol.

**14.** Procédé de coloration des cheveux par oxydation, caractérisé en ce qu'on mélange une quantité suffisante pour la coloration des cheveux d'un colorant capillaire selon l'une des revendications 1 à 8 immédiatement avant l'utilisation avec un oxydant, en particulier de l'eau oxygénée, et on l'applique ensuite aux cheveux, on le laisse agir 10 à 45 minutes à une température de 15 à 50°C, puis on lave les cheveux avec de l'eau et ensuite on les sèche.